Europäisches Patentamt

European Patent Office

Office européen des brevets

(11) Publication number: **0 310 801**

**A1**

## EUROPEAN PATENT APPLICATION

(21) Application number: **88113755.8**

(22) Date of filing: **24.08.88**

(51) Int. Cl.⁴: **A61K 9/10 , A61K 47/00**

(30) Priority: **14.09.87 US 96892**
 **04.08.88 US 227559**

(43) Date of publication of application:
 **12.04.89 Bulletin 89/15**

(84) Designated Contracting States:
 **AT BE CH DE ES FR GB GR IT LI LU NL SE**

(71) Applicant: **ABBOTT LABORATORIES**

**Abbott Park Illinois 60064(US)**

(72) Inventor: **Adjei, Akwete L.**
 **38770 Red Oak Terrace**
 **Wadsworth Illinois 60083(US)**
 Inventor: **Doyle, Richard B.**
 **13 West Hawthorne Drive**
 **Round Lake Beach Illinois 60073(US)**
 Inventor: **Borodkin, Saul**
 **14905 Imperial Drive**
 **Libertyville Illinois 60048(US)**

(74) Representative: **Modiano, Guido et al**
 **MODIANO, JOSIF, PISANTY & STAUB**
 **Modiano & Associati Baaderstrasse 3**
 **D-8000 München 5(DE)**

(54) Anhydrous oil-based liquid suspension for delivering a medicament.

(57) A liquid anhydrous suspension for delivering a medicament, such as clorazepate dipotassium. The suspension contains sugar, oil, a suspending agent, salt, an alkaline buffer, a preservative agent, a surfactant and flavoring.

EP 0 310 801 A1

# ANHYDROUS OIL-BASED LIQUID SUSPENSION FOR DELIVERING A MEDICAMENT

## Technical Field

This is a continuation-in-part of U.S. Serial No. 096,892 filed September 14, 1987.

This invention relates to liquid drug vehicles, and more particularly to liquid oil-based drug vehicles.

## Background Art

Many useful drugs are available only in solid dosage forms (e.g. capsules and tablets), primarily because the drug is unstable in or incompatible with water. For example, clorazepate dipotassium (sold by Abbott Laboratories under the trade name of TRANXENE is one such drug that is not available in liquid form. Clorazepate dipotassium is a minor tranquilizer used for the management of anxiety disorders and for short-term relief of symptoms of anxiety. It is also used as adjunctive therapy in the management of partial seizures and in the symptomatic relief of acute alcohol withdrawal. A description of this drug and its method of manufacture is found in U.S. Patent No. Re. 28,315.

Vehicles for clorazepate dipotassium are unavailable in liquid form primarily because the drug has significant aqueous stability problems. In the presence of even minute quantities of moisture greater than about 0.3% w/v (i.e. 0.3g/100ml) clorazepate dipotassium rapidly hydrolyzes to nordiazepam. This occurs even more rapidly in an acid environment.

Unfortunately, oil-based systems have not been considered suitable as an alternative liquid vehicle for drugs having aqueous stability problems. Although some liquid systems for medicaments are known to contain oil, these are typically formulated to contain water also (e.g. Milk of Magnesia). Even those oil-containing systems not specifically formulated to contain water suffer from the problem that many oils are hygroscopic, and absorb water from the environment. As discussed above, systems containing water would be unsuitable for use with a drug such as clorazepate dipotassium because of the destabilizing effect of water on the drug.

Moreover, even in the absence of water, oil-based systems would be avoided because of the unpleasant taste of oil. Hence, oil-based systems have been considered unacceptable even for drugs not having extreme aqueous stability problems.

## Summary of the Invention

The present invention relates to a liquid suspension for delivering a drug. The suspension is anhydrous, being substantially free of water, and contains oil and suspending agent.

One preferred embodiment also contains a drying agent to help bind water that would otherwise degrade clorazepate dipotassium to nordiazepam. It is also preferred that a buffer be present to stabilize the suspension at a pH favorable to the drug being delivered.

In another preferred embodiment the suspension also contains one or more ingredients such as sugar, salt and flavor to make the suspension palatable.

The suspension of the present invention provides a remarkably stable environment for water-sensitive drugs, such as clorazepate dipotassium. In addition, the suspension is chemically and physically compatible with drugs like clorazepate dipotassium. Furthermore, there is no oily taste or mouthfeel or bitter after taste of the suspension made according to the embodiments containing sugar, salt and flavorings.

The foregoing features and advantages of the present invention will be further understood upon consideration of the following description of the preferred embodiments.

## Description of the Preferred Embodiments

Generally, the suspension of the present invention is an anhydrous oil-based liquid suspension for delivering a medicament. As used herein, the term "anhydrous" means having less than about 0.8% w/v moisture. In the preferred embodiments, the suspension contains less than about 0.3% w/v moisture. In addition to oil, the suspension contains a suspending agent to assure that the medicament is uniformly dispersed within the suspension.

The oil is preferably one that is considered safe for oral consumption, and is relatively stable. Furthermore, the oil should not be incompatible with the drug being delivered. Preferred oils are selected from mixed triglycerides derived from capric, caproic, and caprylic acids. Furthermore, the vegetable oils containing glycerides of aliphatic acids, saturated or unsaturated, such as stearic, palmitic, lauric, margaric, linoleic, linolenic, and myristic acids are suitable for use in the present invention. The most preferred embodiments presently use a winterized safflower oil, available from California Oils, Richmond, California.

The suspending agent functions to suspend the drug uniformly in the oil-based vehicle so that dosages of the drug are uniform. Preferred suspending agents for use in the preferred embodiment of the present invention include bentonite, a finely powdered montmorillonite aluminum silicate, which is available under the tradename Bentone from American Colloid Corporation, and a finely powdered sodium calcium alginate available under the trade name KelsetÄ (from Kelco, a division of Merck & Company) which is also referred to as trihydroxystearin and hydrogenated castor oil available under the tradename Thixcin R (from NL Chemicals, Highstown, New Jersey). Other suspending agents, such as cellulose derivatives, silicates, bentonites, stearates, silicon dioxide and acacia may also be advantageously utilized.

In one preferred embodiment, a drying agent is also included in the suspension. The drying agent is a hygroscopic compound that absorbs or adsorbs water found in the suspension and which may degrade the drug. The drying agent may, in some circumstances, also function as the suspending agent. For example, silica gel has been found to have good suspending properties as well as good water absorbing properties. Silica gel is used as the drying agent in the preferred embodiments of the present invention.

Other preferred embodiments also contain sugar, flavor and salt. These ingredients help to make the otherwise unpalatable oil-based suspension palatable, in view of the unpleasant taste of the oil, and the bitterness of the drug.

Virtually any sweetener that is physically and chemically compatible with the drug being delivered is suitable. The preferred sweetner used is sucrose, and is most advantageously micronized and superfine so as to disperse easily in the suspension. Other sugars, however, such as maltose, fructose, sorbitol and xylitol may also be used.

The preferred flavoring agent is a mixture of vanilla and peppermint extracts, but may also include other oil compatible flavoring agents, such as cherry, chocolate, cinnamon, coconut, coffee, orange, lemon, lime, strawberry, and peanut. In the preferred embodiments the extracts are free of glycol and alcohol, both of which degrade clorazepate dipotassium.

The salt is used primarily as a flavor enhancer to avoid a bland tasting product. The salt also masks the oiliness of the vehicle. It should be present in an amount sufficient to mask the oiliness of the oil based suspension without producing a noticeable salty taste. Sodium chloride is the preferred flavor masking agent. Another suitable masking agent is potassium chloride.

In yet another preferred embodiment, the vehicle contains an alkaline buffer and a preservative. The alkaline buffer maintains the pH at a level at which the drug is stable. Clorazepate dipotassium, for example, is most stable at a pH greater than about 9.0. Potassium carbonate is the preferred buffer; however, other carbonates, hydroxides, oxides and organic amines may be used.

Suitable preservative agents have been found among the paraben class of compounds. Most preferred is a combination of parabens, such as those marketed under the tradename Aseptoform M (methylparaben) and Aseptoform P (propylparaben) available from Mallinckrodt, Inc. of St. Louis, Missouri. Any biologically acceptable benzoate or other preservative system that does not complex with or solubilize the drug to be delivered would also be suitable.

The presence of surfactants has also been found to be advantageous to the physical stability of the suspension. Most surfactants that are compatible with the drug to be delivered as well as the oil vehicle are deemed suitable for use in the present invention. The preferred surfactant is marketed under the tradename Emulphor El 719P by GAF Corp., Wayne, N.J. Emulphor EL 719P is a polyoxyethylated vegetable oil having low dioxin levels.

Generally, the ingredients of the suspension of the preferred embodiments of the present invention should be present in the following amounts:
an effective amount of a medicament,
about 30% w/v to about 75% w/v sugar,
about 30% w/v to about 75% w/v oil,
about 0.05% w/v to about 5% w/v suspending agent,
about 0.05% w/v to about 2% w/v salt,
about 0.01% w/v to about 2% w/v alkaline buffer,
about 0.01% w/v to about 3% w/v preservative,

less than about 5% w/v surfactant, and
about 0.01% w/v to about 5% w/v flavor.

Formulations having less than about 30% w/v sugar were found to be unpalatable, while those having more than about 75% w/v had a consistency that was too thick.

More preferable, the ingredients are present in the following ranges:
an effective amount of a medicament,
about 40% w/v to about 65% w/v sugar,
about 45% w/v to about 65% w/v oil,
about 0.1% w/v to about 2% w/v suspending agent,
about 0.2% w/v to about 1% w/v salt,
about 0.05% w/v to about 1% w/v alkaline buffer,
about 0.1% w/v to about 1% w/v preservative,
less than about 2% w/v surfactant,
about 0.01% w/v to about 3% w/v flavoring.

More advantageously, a suspension containing an effective amount of clorazepate dipotassium contains about 45% w/v sugar, about 52% w/v oil, about 0.75% w/v suspending agent, about 0.3% w/v salt, about 0.1% w/v alkaline buffer, about 0.3% w/v preservative, about 1% w/v surfactant, and about 0.15% w/v flavor. The suspension is substantially free of water and alcohol.

The following examples of anhydrous liquid suspensions for clorazepate dipotassium serve to illustrate the preferred embodiments of the present invention.

## Example 1

320g Neobee oil was combined with 5g Bentone 34 and 290g. sugar. 1.84g of sodium chloride powder was then added. To 100 ml of the above mixture was added 150mg clorazepate dipotassium. Ingredients were mixed well. The resulting formulation tasted slightly saline, moderately sweet, with an oily and slightly bitter aftertaste.

## Example 2

To 244g of the mixture of Example 1 was added 4g surfactant PVP K30 (polyvinyl pyrolidone having an average molecular weight of about 30,000). The resulting mixture was smooth, thin, and free flowing. To this mixture was then added 0.5g vanilla flavor. The taste was good, but the mixture turned very thick. An additional 244g of the mixture of Example 1 was added to thin out the suspension. The resulting mixture was thick but pourable and had excellent taste.

## Example 3

375mg clorazepate dipotassium was mixed with 250ml vehicle produced from Example 2. Taste was excellent, but some bitterness of the drug was noted.

## Example 4

300g Neobee oil M5, 300g powdered sugar 12X and 5.0g Bentone 34 were combined. Some evidence of thixotropy was noted at final volume. 1.0g vanilla flavor was added, causing the suspension to thicken significantly. The ingredients were mixed well at high speed for 15 minutes.

An additional 70.35g Neobee oil M5 was added to 2.0g sodium chloride which had been reduced to a fine powder in mortar/pestle before addition to suspension. In order to formulate a suspension containing 7.5mg drug per 5ml of suspension, 300mg of clorazepate dipotassium was added to 200ml vehicle produced above and then mixed well. The resulting mixture was heavy, thixotropic and tasted good.

## Example 5

600g Neobee oil M5 and 8.85g Bentone 34 were mixed together well. 3.60g powdered sodium chloride was added with good mixing. 517g powdered sugar 12X was added and mixed until smooth. The resulting mixture was allowed to deaerate. An additional 34g Neobee M5 oil was then added to bring the volume of the mixture to 1.0 liter, and then mixed until uniform. A small amount of vehicle was removed and mixed with 1.50g clorazepate dipotassium until smooth, and then added back into the main batch and mixed well. 1.80g vanilla flavor was added. The mixture became thixotropic after the addition of vanilla. The taste of the suspension was good.

## Example 6

To 6.30kg Neobee M5 oil was mixed 86.0g Sentone 34. 10.0g powdered potassium carbonate and 35.0g powdered sodium chloride were mixed with above oil mixture to make a smooth paste. The paste was diluted with additional oil and mixed until uniform consistency was attained. The diluted paste was then added to the main batch. Clorazepate dipotassium was mixed with a small amount of oil until smooth, and then diluted further and added to the main batch. 5.20kg powdered sugar 12X was slowly added to the main batch and mixed for approximately 1 hour. Immediately after all the sugar was added the suspension showed thixotropic character. After 1 hour of mixing the mixture became smooth and uniform, and lost its thixotropic character, 15ml flavor was then added. 100g Emulphor El 719P surfactant was added to thicken the mixture. The mixture was passed through a Manton & Gaulin homogenizer at 1500 psi to yield a smooth and uniform product. The product was placed in a vacuum for 1 hour to deaerate. The taste of the suspension was good.

## Example 7

From an initial amount of 1833g Neobee oil, 1 liter Neobee oil was taken and mixed with 39g Emulphor EL 719P. 25g Bentone 34 was blended with Neobee oil and added to the main batch. 10g powdered sodium chloride was blended with a small amount of Neobee oil and then added to the main batch. A preservative agent having 6.8g Aseptoform M and 3.4g Aseptoform P was mixed in 40g of oil and heated until completely in solution, and then added to the main batch.

The balance of the oil was then added and blended with a small amount of oil and added to the main batch. 4.46 clorazepate dipotassium was blended with a small amount of oil and added to the main batch. The balance of the oil was added and blended. 1513.0g powdered sugar was mixed in and deaerated. 4.5ml natural and artificial vanilla/peppermint extract (obtained from Florsynth, Des Plaines, Illinois) was also mixed in. The entire mixture was passed through a Manton & Gaulin homogenizer. The taste of the suspension was good.

## Example 8

A placebo vehicle was prepared by mixing a small amount of 6.0kg of Neobee 5M oil with 85.5 of Bentone 34. The Bentone 34/Neobee oil mixture was then added to the balance of the Neobee oil. 6.17kg powdered sugar 12X was mixed in. 36.00g powdered sodium chloride was mixed in. The resulting mixture was thick after the first hour of mixing with a turbine blade, but thinned out after 3 hours mixing. The taste of the suspension was good.

Example 9

A prototype stability batch was prepared by mixing 2.95kg safflower oil with 5.0g Aseptoform M and 1.0g Aseptoform P. The mixture was heated to approximately 80°C and 45.0g and 12.5g Thixcin-R were added. After dissolving the ingredients, the mixture was cooled to room temperature, and vacuum placed on the vehicle. 7.5g clorazepate dipotassium and 10g potassium carbonate were then added while the vehicle was being mixed under vacuum. 17.5g sodium chloride and 3.0kg of powdered sugar were added and homogenized using a Fryma MZM-Vk7 Delmix mixer while still under vacuum. 7.5g flavor was then added and mixed for approximately 15 minutes. The resulting mixture was milled using a Fryma MS-18 Coball mill (obtained from Fryma, Inc., 40 Ethel Road, Edison, N.J.) and found to have acceptable taste and stability requirements.

Example 10

| Ingredients | % w/v | Amt/Ltr |
|---|---|---|
| Neobee M5 Oil | 44.00% | 480.00 gm |
| Aseptoform M powder | 0.20% | 2.00 gm |
| Aseptoform P powder | 0.02% | 0.20 Gm |
| Sodium Chloride | 0.35% | 3.50 Gm |
| Kelset | 0.90% | 9.00 Gm |
| Thixcin-R | 0.15% | 1.50 Gm |
| Cab-O-Sil | 0.50% | 5.00 Gm |
| Tween 80 | 2.00% | 20.00 Gm |
| Powdered Sugar, 12X | 48.00% | 480.00 Gm |
| Iron Oxide | 0.01% | 0.10 Gm |
| Chocolate | 0.05% | 0.50 Gm |
| Flavor Enhancer, Prosweet | 0.75% | 7.50 Gm |
| Vanilla Peppermint Flavor | 0.15% | 1.50 Gm |
| Estazolam | 0.02% | 0.20 Gm |
| Neobee M5 Oil | q.s. | q.s. |

400 ml Neobee M5 oil was heated to approximately 90°C and then allowed to stand for approx. 1 hour to equilibrate. Aseptoform M and P powders were mixed in till dissolved. Kelset, Thixcin, Tween 80 were mixed to form an uniform dispersion. Estazolam was added and homogenized in a beadmill or a high energy mill. Flavors were then added until the resulting product was uniform. The volume of the resulting products was adjusted to 1 liter with Neobee Oil if necessary.

Although the present invention has been described in connection with presently preferred embodiments, those skilled in the art will recognize many modifications to sequence, arrangement, portions, elements, and materials which can be used in the practice of the invention without departing from its scope. It is intended that such changes and modifications be covered by the following claims.

**Claims**

1. A liquid anhydrous suspension for delivering a medicament, said suspension comprising a medicament, an oil and a suspending agent.

2. The liquid anhydrous suspension of claim 1, further comprising a drying agent; one or more of sugar, salt and flavor, such that the suspension is palatable; a buffer; a surfactant; and a preservative.

3. The suspension of claim 1 wherein the medicament is clorazepate dipotassium.

4. An anhydrous liquid suspension for delivering a medicament comprising:
an effective amount of a medicament;
about 30% w/v to about 75% w/v oil; and
about 0.05% w/v to about 5% w/v/ suspending agent.

5. The suspension of claim 4, further comprising:

about 30% w/v to about 75% w/v sugar;

about 0.05% w/v to about 2% w/v salt;

about 0.01% w/v to about 2% w/v buffer;

about 0.01% w/v to about 3% w/v preservative; and

about 0.01% w/v to about 5% w/v flavor.

6. The suspension of claim 5, wherein

said sugar is present in an amount of about 40% w/v to about 65% w/v;

said oil is present in an amount of about 40% w/v to about 65% w/v;

said suspending agent is present in an amount of about 0.1% w/v to about 2% w/v;

said salt is present in an amount of about 0.2% w/v to about 1% w/v;

said buffer is present in an amount of about 0.2% w/v to about 1% w/v;

said preservative is present in an amount of about 0.1% w/v to about 1% w/v; and

said flavor is present in an amount of about 0.1% w/v to about 3% w/v.

7. The suspension of claim 6, wherein the medicament is clorazepate dipotassium.

8. An anhydrous liquid suspension for delivering a medicament comprising:

a sugar present in an amount of about 520 g/l;

an oil present in an amount of about 630 g/l;

a suspending agent present in an amount of about 8.6 g/l;

a salt present in an amount of about 3.5 g/l;

a buffer present in an amount of about 1 g/l;

a preservative present in an amount of about 2 g/l of methyl paraben and about 1 g/l of propyl paraben;

a flavor present in an amount of about 1.5 g/l; and

a pharmaceutically effective amount of clorazepate dipotassium.

9. The suspension of claim 8 wherein the sugar is confectionary sugar; the oil is safflower oil; the suspending agent is sodium calcium alginate; the salt is sodium chloride; and the buffer is potassium carbonate.

10. The suspension of claim 9 wherein the clorazepate dipotassium is present in an amount of about 1.5 to 3.0 g/l.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | US-A-4 080 445 (SONG-LING LIN et al.) <br> * Column 2, line 29 - column 4, line 17 * <br><br> --- | 1,4 | A 61 K 9/10 <br> A 61 K 47/00 |
| X | US-A-2 951 014 (H.G. GARMAN) <br> * Column 2, lines 14-30; example 3; claims 1-10 * <br><br> --- | 1,4 | |
| A | US-A-3 920 819 (V.C. STEPHENS et al.) <br> * Column 2, line 1 - column 3, line 40 * <br><br> --- | | |
| A | DE-A-2 250 680 (ANTONIO GALLARDO SA) <br> * Page 1, line 1 - page 2, line 16 * <br> ----- | | |

TECHNICAL FIELDS SEARCHED (Int. Cl.4)

A 61 K

The present search report has been drawn up for all claims

| Place of search | Date of completion of the search | Examiner |
|---|---|---|
| THE HAGUE | 02-02-1989 | TZSCHOPPE,D.A. |

EPO FORM 1503 03.82 (P0401)